Europäisches Patentamt

**European Patent Office**

Office européen des brevets

(19)

(11) Numéro de publication: **0 287 470 B1**

(12)

# FASCICULE DE BREVET EUROPEEN

(45) Date de publication de fascicule du brevet: **24.02.93**   (51) Int. Cl.5: **A61K 35/30**, B01D 15/08

(21) Numéro de dépôt: **88400909.3**

(22) Date de dépôt: **14.04.88**

(54) **Application des résines constituées par des polymères fonctionnels comme phase stationnaire en chromatographie d'affinité pour la purification des facteurs de croissance et procédé de purification correspondant.**

(30) Priorité: **17.04.87 FR 8705546**

(43) Date de publication de la demande:
**19.10.88 Bulletin 88/42**

(45) Mention de la délivrance du brevet:
**24.02.93 Bulletin 93/08**

(84) Etats contractants désignés:
**AT BE CH DE ES FR GB GR IT LI LU NL SE**

(56) Documents cités:
**EP-A- 0 216 668
FR-A- 2 573 997
US-A- 4 444 760**

**PROCEEDINGS OF THE NATIONAL ACADEMICAL SOCIETY, vol. 81, janvier 1984, Biochemistry; K.A.THOMAS et al., pp. 357-361.**

(73) Titulaire: **CENTRE NATIONAL DE LA RECHERCHE SCIENTIFIOUE**
**15, quai Anatole France**
**F-75007 Paris(FR)**

(72) Inventeur: **Barritault, Denis Stéphan Charles**
**4 rue Française**

**F-75001 Paris(FR)**
Inventeur: **Badet, Josette née Genissel**
**20 rue des Crocheteurs**
**F-92160 Antony(FR)**
Inventeur: **Courty, José Patrice**
**15 Allée verte**
**F-94440 Villecresnes(FR)**
Inventeur: **Dourges, Marie-Anne née Jacquot**
**12 Clos St Pierre**
**F-95480 Pierrelaye(FR)**
Inventeur: **Gulino, Danielle née Debrac**
**4 Allée des Jonquilles**
**F-93360 Neuilly Plaisance(FR)**
Inventeur: **Jozefonvicz, Jacqueline née Dorgebray**
**65 Deuxième Avenue**
**F-60260 Lamorlaye(FR)**

(74) Mandataire: **Phélip, Bruno et al**
**c/o Cabinet Harlé & Phélip 21, rue de La Rochefoucauld**
**F-75009 Paris (FR)**

EP 0 287 470 B1

Rank Xerox (UK) Business Services
(3.10/3.5x/3.0.1)

**Description**

La présente invention concerne la purification des facteurs de croissance par chromatographie d'affinité sur des résines porteuses de groupes capables de leur conférer une affinité sélective vis-à-vis de ces facteurs de croissance.

Dans la littérature, on a décrit des activités facteurs de croissance qui se définissent comme des extraits contenant des composés capables d'agir sur la migration et la prolifération cellulaires, notamment en jouant un rôle important dans la néovascularisation des tissus sains ou pathologiques. Ces activités facteurs de croissance sont obtenues à partir d'un grand nombre de tissus, notamment à partir de l'hypophyse, du cerveau, de l'hypothalamus, de la rétine et de différents tissus oculaires, du cartilage et des chondrosarcomes, et, également, du rein, du foie, du placenta, du corpus lutéum, des muscles, etc. Il ressort de nombreux travaux de recherche que, parmi ces facteurs, ceux qui peuvent être purifiés par chromatographie d'affinité sur Heparin Sepharose, appartiennent à deux groupes ayant ces activités. Ces protéines ont été séparées et caractérisées et leur structure primaire a été élucidée.

Le premier groupe de ces protéines a été décrit sous les noms de FGF (ou basic FGF), AGF2 (astroglial growth factor), EDGFI, $\beta$-HBGF (bêta heparin binding growth factor) BDGF$_1$ (bain derived growth factor) ; quant au deuxième, il a été décrit sous les noms de ECGF, acidic FGF, AGF1, EDGFII, RDGF (retinal derived growth factor), $\alpha$-HBGB, BDGF$_2$. Par simplification, ces facteurs de croissance connus seront dénommés bFGF et aFGF. Les facteurs de croissance aFGF présentent un point isoélectrique compris entre environ 5 et 5,8 et une masse moléculaire comprise entre 16000 et 18000 daltons. Quant aux facteurs de croissance bFGF, ils présentent un point isoélectrique de l'ordre de 9 à 10 et une masse moléculaire comprise entre 16000 et 19000 daltons. A titre de référence bibliographique concernant ces facteurs de croissance, on citera R. R. LOBB et al, "Analytical Biochemistry 154, 1-14 (1986)" ainsi que J. Courty et al, dans "Biochem Biophys. Res. Commun., 136, 102-108 (1986)".

Ainsi qu'on l'a mentionné ci-dessus, les facteurs de croissance de type FGF sont purifiés par chromatographie d'affinité sur des résines à base de polysaccharides sur lesquels est fixée de l'héparine. A titre d'exemple de résines de ce type, on peut mentionner celle qui est commercialisée sous la dénomination "Heparin Sepharose" par la Société "PHARMACIA". Sur cette résine, les facteurs aFGF sont élués avec un tampon pH neutre, d'une force ionique équivalente à celle d'une solution de NaCl voisine de 1M, et les facteurs bFGF sont élués avec un tampon pH neutre, d'une force ionique équivalente à celle d'une solution de NaCl comprise entre 1,5 et 2,0M. Ainsi, après mise en contact avec la résine "Heparin Sepharose" de l'extrait brut contenant les facteurs de croissance, l'élution de ceux-ci est réalisée avec un gradient de chlorure de sodium par exemple.

Le support chromatographique précité, à base d'héparine, est un gel qui ne permet qu'une utilisation en basse pression, ce qui limite considérablement son emploi à l'échelle industrielle. De plus, l'héparine étant un polysaccharide très hétérogène (soit un mélange de glycosaminoglycanes extraits de l'intestin de porc et présentant une large distribution de poids moléculaires), les purifications chromatographiques utilisant la résine "Heparin Sepharose" ne sont pas toujours reproductibles et dépendent du lot d'héparine qui a été couplé sur la résine de base.

Pour pallier ces inconvénients, on a recherché, pour cette application, des supports totalement synthétiques dont les propriétés biologiques sont analogues à celles de l'héparine, qui possèdent de bonnes propriétés mécaniques pour permettre la purification de ces facteurs de croissance en haute pression, et qui peuvent résister aux contraintes chimiques imposées par leur régénération après utilisation en chromatographie.

On a alors découvert une famille de polymères fonctionnels bien définis qui répond au problème posé et qui peut ainsi remplacer avantageusement la résine "Heparin Sepharose" pour la purification de facteurs de croissance de type FGF par chromatographie liquide.

Ces résines sont constituées par des polymères ou copolymères sur lesquels sont fixés, de façon statistique, des groupements -SO$_3$H et -SO$_3$M (où M représente un métal physiologiquement acceptable), ainsi que, de préférence, également des groupes de formule -SO$_2$R (où R représente un radical obtenu par enlèvement d'un atome d'hydrogène sur la fonction amine d'un acide aminé ou d'un dérivé d'acide aminé). Ces groupes chimiques sont avantageusement fixés de façon irréversible par réticulation.

La présente invention porte donc d'abord sur l'utilisation de cette famille de résines comme phase stationnaire en chromatographie d'affinité pour la purification des facteurs de croissance.

Le support polymère ou copolymère de base utilisé est choisi notamment parmi le polystyrène, les polysaccharides, les polyesters cellulosiques, les polyéthers cellulosiques, le poly(acétate de vinyle) le poly(chlorure de vinyle), le polyisoprène, le polybutadiène, et les copolymères du styrène, des esters cellulosiques, des éthers cellulosiques, de l'acétate de vinyle, du chlorure de vinyle, de l'isoprène et du butadiène.

On peut mentionner le polystyrène comme polymère particulièrement intéressant.

Par ailleurs, les polymères ou copolymères utilisés sont avantageusement des produits réticulés.

Il n'y a pas de limitation particulière en ce qui concerne les acides aminés, en particulier $\alpha$-aminés, et leurs dérivés, devant conduire au radical R précité. On pourra mentionner, à titre d'exemples, notamment l'alanine, la phénylalanine, les diacides aspartique et glutamique, la méthionine, la thréonine, la proline, l'hydroxyproline, la sérine et la lysine.

Parmi les acides aminés autres que les acides alpha-aminés, on peut mentionner l'acide aminocaproïque, l'acide aminovalérique et l'acide aminobutyrique.

Les résines qui sont utilisées pour la mise en oeuvre de l'invention sont déjà connues ; elles ont notamment été décrites dans le brevet français n° 2 461 724.

Leur procédé de préparation comprend une première étape consistant à faire réagir de l'acide chlorosulfonique sur le polymère choisi, dans un solvant approprié, de façon à obtenir un polymère chlorosulfoné (c'est-à-dire portant des groupes -$SO_2Cl$), et une deuxième étape consistant soit à hydrolyser ledit polymère chlorosulfoné avec une base MOH pour transformer les groupes -$SO_2Cl$ en groupes -$SO_3M$, soit à faire réagir ledit polymère chlorosulfoné avec l'acide aminé choisi, dans le milieu basique MOH, pour transformer les groupes -$SO_2Cl$ en groupes -$SO_3M$ et en groupes -$SO_2R$.

La présente invention porte également sur un procédé d'obtention d'un facteur de croissance aFGF ou bFGF purifié, ou d'un mélange de ces facteurs de croissance, caractérisé par le fait qu'il consiste à effectuer une chromatographie d'affinité, sur une résine telle que définie ci-dessus. On peut effectuer une chromatographie sous haute pression. Egalement, la chromatographie d'affinité peut être conduite sur plusieurs résines en série. On réalise une élution notamment avec un tampon pH neutre d'une force ionique équivalente à celle d'une solution de NaCl 0,15M à 2,5M.

Pour illustrer davantage l'objet de la présente invention, on va en décrire maintenant, à titre d'exemples illustratifs, plusieurs modes de réalisation.

En premier lieu, on décrira la préparation des différentes résines modifiées utilisées.

Le polymère de départ était un polystyrène consistant en un copolymère de styrène et de divinylbenzène (98/2 en poids), commercialié par BIORAD. Il se présentait sous forme de billes d'un diamètre compris entre 40 et 80 $\mu$m environ. Le produit commercial a été lavé successivement avec une solution 1M de NaOH, de l'eau, une solution 1M de HCl et de l'eau. Il a été séché sous vide à 60°C.

Les acides aminés utilisés étaient des réactifs "FLUKA PURISS".

A) - Préparation du PS-$SO_3Na$

On laisse gonfler une nuit, à la température ambiante, 25 g de polystyrène dans 200 ml de dichlorométhane. On ajoute alors un mélange de 160 ml de chlorure de méthylène et de 140 ml d'acide chlorosulfonique. La suspension est agitée pendant 4 heures, à 40°C. La résine brute est alors filtrée, lavée avec précaution avec du chlorure de méthylène et de l'acétone. Elle est finalement séchée sous vide à 50°C.

Le taux de groupes chlorosulfonyle est déterminé de la façon suivante : 200 mg de polystyrène chlorosulfoné sont hydrolysés avec 50 ml d'une solution 1M de NaOH pendant 24 heures au reflux. Après acidification, les ions Cl$^-$ sont titrés par une solution 0,1M de $AgNO_3$ en utilisant une électrode indicatrice d'argent.

Le polystyrène sulfoné est hydrolysé quantitativement par la soude 2M à la température ambiante. On filtre, on lave à l'eau et on sèche sous vide.

B) Préparation du PS-$SO_2R$

On utilise une quantité de l'acide aminé choisi correspondant à deux moles par groupe chlorosulfoné. On dissout l'acide aminé dans 125 ml du mélange 3:2 eau-dioxanne, par addition du minimum de soude 4M. Le pH est mesuré et on ajoute alors 10 g du polystyrène chlorosulfoné tel qu'obtenu ci-dessus (43 méq-$SO_2Cl$). Le pH est ensuite maintenu à sa valeur initiale par addition de soude 0,5M. La réaction est arrêtée quand le pH reste stable. Le polymère est alors filtré, lavé abondamment avec de l'eau, puis avec de la soude $10^{-2}$M et de l'eau. Il est enfin séché sous vide.

Les conditions de synthèse des résines utilisées pour la mise en oeuvre des exemples de l'invention sont résumées dans le Tableau I ci-après.

TABLEAU I

| Résine modifiée | Acide aminé | Poids de l'acide aminé (g) | Temps de réaction (mn) | NaOH 2M initial (ml) | NaOH 0,5M (ml) | pH apparent |
|---|---|---|---|---|---|---|
| PSAla | Alanine | 7,5 | 47 | | 54 | 11 |
| PSPhé | Phényl-alanine | 13,9 | 37 | 50 | 33,5 | 11,6 |
| PSGlu | Acide glutamique | 12,4 | 67 | 86 | 21 | 11,1 |
| PSAsp | Acide aspartique | 11,2 | 100 | 91,5 | 25,5 | 11,1 |
| PSMéth | Méthionine | 12,5 | 16 | 53 | 20 | 11,2 |
| PSThréo | Thréonine | 9,3 | 36 | 45 | 27 | 11,5 |
| PSSer | Sérine | 8,4 | 58 | 46 | 32 | 11,6 |
| PSPro | Proline | 9,2 | 14 | 41 | 35,5 | 11,5 |

Poids PS $SO_2Cl$ = 10 g, sauf dans le cas de la préparation de PSThréo où ce poids est de 9,3 g

Dans certains cas, notamment si l'on utilise l'acide aspartique, il est préférable de fixer, sur le polyester, le diméthylester de l'acide correspondant, en faisant suivre par une hydrolyse basique. La fixation de l'ester se fait en milieu dichlorure de méthylène en présence de triéthylamine ($Et_3N$). Les conditions opératoires sont résumées dans le Tableau II

TABLEAU II

| Résine modifiée par l'ester diméthylique de l'acide aspartique | Temps de réaction (h) |
|---|---|
| PSAsp(OM)$_2$-a | 84 |
| PSAsp(OM)$_2$-b | 15 |
| PSAsp(OM)$_2$-c | 6 |

Poids PS$SO_2Cl$ : 7 g

Poids de l'ester diméthylique de l'acide aspartique : 9 g

Volume $CH_2Cl_2$ : 250 ml

Volume $Et_3N$ : 10,3 ml

Temps d'hydrolyse : 24 h

Température d'hydrolyse : 40°C

4

Les compositions chimiques de ces polymères sont récapitulées dans le Tableau III suivant :

TABLEAU III

| Résines modifiées | Dosage acidi-métrique (méq/g) | Dosage par microanalyse (méq/g | % Motifs d'acide aminé | % Motifs de $SO_3$ |
|---|---|---|---|---|
| PSAla | 3,1 | 1,5 | 30,7 | 46,6 |
| PSPhé | 0,60 | 0,76 | 15,2 | 62,2 |
| PSGlu | 0,11 | 0,14 | 2,8 | 74,6 |
| PSAsp | 0,35 | 0,34 | 7 | 70,4 |
| PSMéth | 0,51 | 0,68 | 13,5 | 63,9 |
| PSThréo | 0,57 | 0,86 | 16 | 61,4 |
| PSSer | 0,94 | 0,76 | 14,3 | 65,7 |
| PSPro | 0,95 | 1,03 | 20,9 | 59,1 |
| PSAsp(OM)$_2$-a | | 2,42 | 74,3 | 11,7 |
| PSAsp(OM)$_2$-b | | 2,57 | 73,8 | 15,2 |
| PSAsp(OM)$_2$-c | 2,35 | 2,43 | 73 | 16 |
| PSHyPro | | | 24 | 56 |
| PSSO$_3$Na | | | 0 | 90 |

Dans ce qui suit, on décrira, à titre illustratif, l'obtention de l'extrait acido-soluble de cerveau contenant les facteurs de croissance aFGF et bFGF selon la technique décrite dans la demande de brevet français FR-A-2 600 655.

Toutes les opérations ont lieu à 4°C. 4 kg de tissus sont homogénéisés à l'aide d'un broyeur Turax dans 1 litre de tampon PBS. Les débris cellulaires sont éliminés par centrifugation à 10000 g, pendant 45 mn (Rotor Benckmann JA 10). Le surnageant, appelé extrait brut, est ajusté à une concentration de 20% $(NH_4)_2 SO_4$ et centrifugé pendant 45 minutes à 10000 g. Le deuxième surnageant est passé sur laine de verre, afin d'éliminer les traces de lipides, puis ajusté à une concentration de 60% $(NH_4)_2SO_4$ et centrifugé à à 10000 g pendant 45 minutes. Le précipité obtenu est redissous dans un volume minimum de PBS (environ 400 ml) et la solution est ajustée à pH 3,2 avec de l'acide acétique glacial, puis immédiatement centrifugée pendant 30 minutes à 10000g. Le surnageant est réajusté à pH 7 avec de la soude concentrée et dialysée extensivement contre du PBS. La solution est ensuite centrifugée pendant 30 minutes à 20000 g (rotor Benckmann JA20), afin d'éliminer toute trace de précipité. Ce dernier surnageant est appelé extrait acido-soluble.

On a ensuite réalisé différentes chromatographies d'affinité sur les résines conformes à l'invention, telles que préparées ci-dessus.

Pour faciliter le suivi de la purification, une petite quantité de aFGF (ou de bFGF) purifié et marqué à l'iode radio-actif ($^{125}$I) (aFGF* ou bFGF*) est ajoutée à l'extrait acido-soluble. On peut donc suivre la purification en déterminant la radioactivité éluée dans les fractions en sortie de colonne.

Il a été vérifié au préalable que les facteurs aFGF* et bFGF* se comportent comme leurs homologues natifs en chromatographie d'affinité. Ces expériences ont été faites sur la résines classique "Heparin Sepharose".

Le premier type d'expérience a consisté à suivre la radioactivité éluée à partir de mélanges composés d'extrait acido-soluble et de aFGF* ou bFGF*.

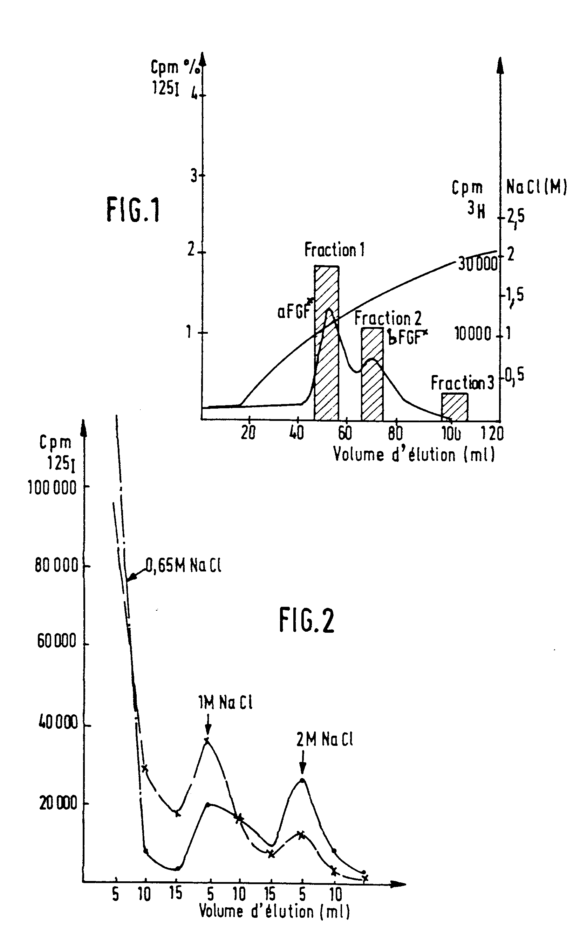

Le deuxième type d'expérience a consisté à suivre la purification des aFGF et bFGF à partir de l'extrait acido-soluble en mesurant l'activité biologique des fractions éluées, en l'absence de facteur marqué à l'iode radioactif. Les tests biologiques utilisés ont consisté à révéler la présence du facteur de croissance en étudiant la prolifération, par incorporation de thymidine tritiée, de cellules cibles induites par les fractions chromatographiques. Comme le montre la figure 1, qui donne le chromatogramme des aFGF et bFGF à partir de l'extrait acido-soluble avec utilisation d'un gradient de NaCl de 150 ml, les fractions riches en radioactivité contiennent l'activité biologique. Il en résulte que les facteurs aFGF* et bFGF* constituent de bons traceurs des deux activités biologiques (Sur la figure, 1, ▨ représente l'activité biologique des fractions étudiées ($^3$H cpm)).

On décrira maintenant les tests chromatographiques qui ont été effectués.

1 g de résine de l'invention est mis à gonfler pendant 15 mn dans 10 ml de tampon PBS et coulé en colonne IBF 11 (10 ml), à raison de 0,5 ml/mn. 1 ml de l'extrait acido-soluble de cerveau qui contient 4 mg de protéines, soit 4 mg par g de résine, est déposé sur la colonne. Les protéines retenues sont éluées successivement avec des solutions de PBS.

I - Tests réalisés par paliers de force ionique :

Ces tests chromatographiques ont été réalisés sur les résines PSAsp et PSSer. Ils ont été effectués en faisant des élutions par paliers successifs de force ionique 0,65M, 1M, 2M NaCl.

Ces tests font apparaître une rétention, puis une élution partielle du bFGF, lorsque ces deux résines sont utilisées comme gel chromatographique. C'est ce qui ressort de la figure 2 et du Tableau IV suivant :

TABLEAU IV

| Résines | % bFGF non retenu | % bFGF* élué | % bFGF* adsorbé définitivement |
|---------|-------------------|--------------|-------------------------------|
| PS Asp  | 27                | 13           | 59                            |
| PS Ser  | 30                | 20           | 50                            |

La figure 2 illustre l'élution d'un mélange bFGF* + extrait acido-soluble, sur PSSer (-o-) our sur PSAsp (-x--x-).

Le dosage des concentrations en protéines totales (méthode de Bradford), parallèlement à la détermination de la radioactivité dans les fractions éluées, permet d'estimer un enrichissement en facteur de croissance. Cet enrichissement est compris entre 10 et 20 pour les fractions éluées avec NaCl 1M ou 2M.

II - Tests utilisant un gradient linéaire

Dans cette série d'essais, dont les résultats sont rapportés dans le Tableau V suivant, seule l'affinité avec le facteur bFGF a été étudiée.

6

A partir de ce Tableau V, on constate que les capacités d'adsorption sont différentes, comparées à celles de l'Heparin Sepharose, testée à titre de comparaison.

En effet, 30% du facteur bFGF sont adsorbés réversiblement sur la résine Héparin Sepharose, alors que 10% et 19% le sont respectivement sur les résines PS Thréo et PS Glu (II) dans des conditions identiques. L'adsorption irréversible du facteur bFGF est relativement importante, quelle que soit la résine testée (29% pour la résine "Heparin Sepharose"), sauf la résine PS Thréo. L'adsorption irréversible non

## TABLEAU V

| Résines | % bFGF[*] | | | Molarité de NaCl Elution bFGF (M) | % Protéines Résines | | | Enrichissement |
|---|---|---|---|---|---|---|---|---|
| | non adsorbé | adsorbé réversible | adsorbé irréversible | | non adsorbé | adsorbé réversible | adsorbé irréversible | |
| Heparin Sepharose | 30 | 30 | 29 | 1,5 | 62,5 | 1,5 | 36 | 20 |
| PS Thréo | 80 | 10 | 1 | 1,2 | 90,6 | 0,4 | 9 | 40 |
| PS Glu (I) | 24 | 23 | 43 | 1,2 | 75,3 | 4,7 | 25 | 5 |
| PS Glu (II) | 36 | 19 | 41 | 1,1 | 79 | 0,3 | 21 | 63 |
| PS Asp | 37 | 13 | 43 | | 80,9 | 0,9 | 18 | 14 |
| PS Asp (OM)$_2$ | 63 | 5 | 29 | 0,9 | 89,2 | 1,1 | 11 | 4,5 |
| PS Phé | 29 | 17 | 53 | 1,3 | 89,1 | 1,6 | 9 | 11 |
| PSSO$_3$ | 20 | 14 | 62 | 1,7 | 69,8 | 1,8 | 28 | 8 |

Poids de résine par colonne = 1 g

I, II : essais chromatographiques successifs sur une même colonne.

spécifique des protéines totales est nettement plus forte dans le cas de la résine Heparin Sepharose.

Il a été observé, après utilisation répétée des colonnes d'Heparin Sepharose, une saturation progressive des sites actifs, s'accompagnant d'une diminution très sensible de l'affinité de ces résines pour les facteurs de croissance. Toutefois, une purification sur une même colonne de résines conformes à l'invention favorise un meilleur enrichissement du bFGF, c'est ce qui a été observé dans le cas de la résine PS Glu (Tableau V).

Ce tableau permet aussi de constater que l'intensité des interactions mises en jeu entre le facteur bFGF et les différentes résines demeure assez voisine, quelle que soit la résine expérimentée. En effet, la force ionique nécessaire à l'élution du facteur bFGF est comprise entre 0,9 et 1,7M NaCl.

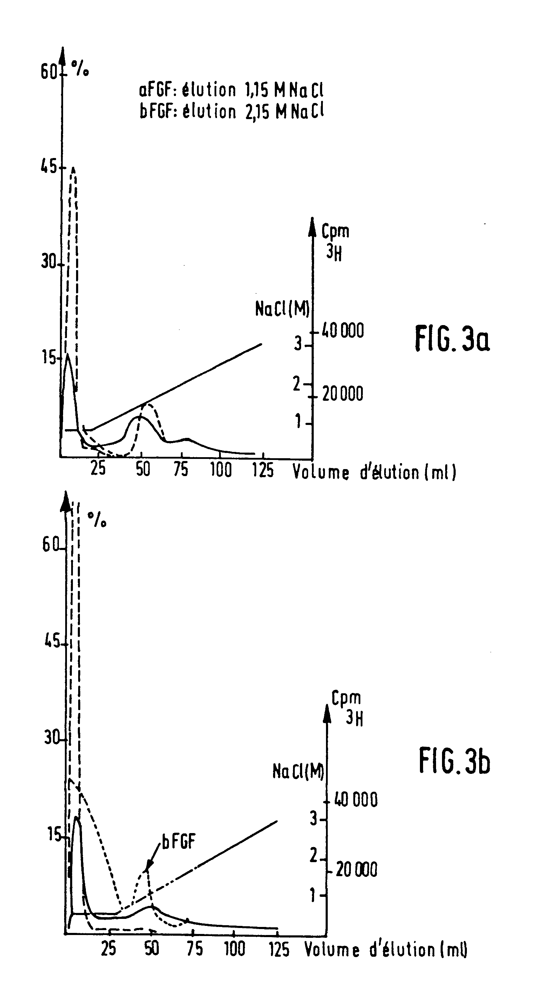

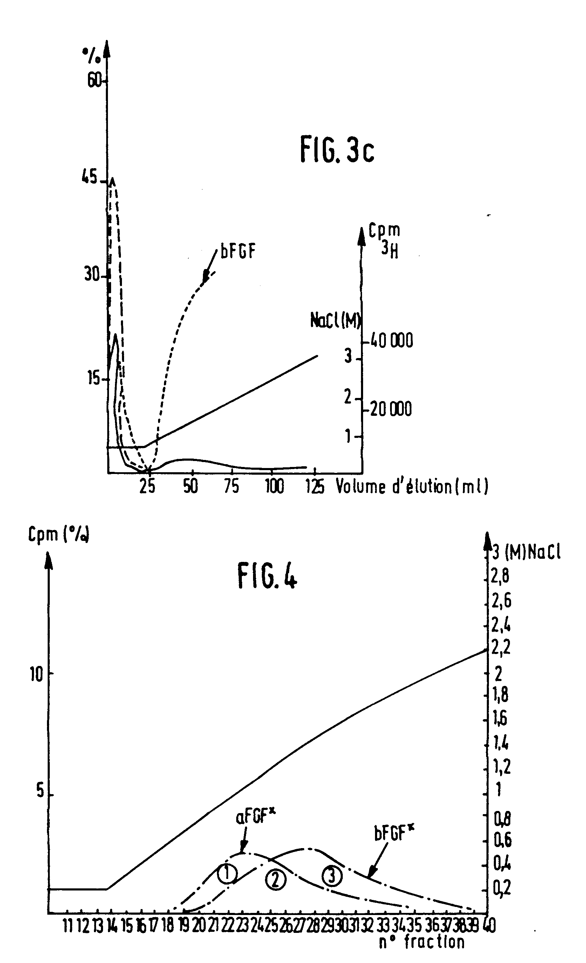

Par ailleurs, parallèlement à l'étude de la radioactivité éluée, des tests d'activité biologique (par incorporation de thymidine tritiée) ont été effectués pour les résines Heparin Sepharose, PSGlu I, PsPhé. Les résultats sont présentés sur la Figure 3.

Légende de la figure 3 :

Chromatographie sur Heparin Sepharose et sur résines de l'invention d'un mélange d'extrait acido-soluble et de bFGF* en utilisant un gradient d'élution de NaCl.
a) Heparin Sepharose ; b) PSGlu ; c) PSPhé

— — — :      bFGF* ($^{125}$ I cpm)
- - - :      concentration protéique
...... :      activité biologique ($^3$H cpm)
           bFGF = élution 2,15M NaCl
           aFGF = élution 1,15M NaCl

En ordonnées, on a porté les % de radioactivité $^{125}$I détectée et également les % de protéines détectées. Les trois types de courbes ont été normées.

Sur ces résines, bien qu'une partie de l'activité biologique soit éluée avec la fraction de protéines non adsorbées, une partie de l'activité biologique est cependant désorbée à l'aide du gradient de NaCl et elle est éluée approximativement au même volume que la radioactivité correspondant au facteur bFGF*. La molarité en NaCl nécessaire à la désorption de l'activité biologique est de 1,6M pour l'Heparin Sépharose, de 1,2M pour les deux autres résines selon l'invention. Dans le cas de la résine PsPhé, l'activité biologique récupérée au cours du gradient est largement supérieure à celle récupérée dans les mêmes conditions sur Heparin Sepharose.

III - Tests utilisant un automate.

Un système de chromatographie automatisée a été mis au point permettant une bonne reproductibilité du gradient de NaCl. L'affinité du facteur bFGF et du facteur aFGF pour les résines mentionnées dans le Tablau VI a été testée dans ces conditions. La figure 4 montre que, dans le cas de la résine PSPhé, les facteurs bFGF et aFGF sont légèrement séparés.

Légende de la figure 4 :

Chromatographie sur PSPhé de mélanges constitués d'extrait acido-soluble et soit de bFGF*, soit de aFGF*, en utilisant un gradient d'élution de NaCl.
- ——— bFGF* et aFGF*

L'analyse des fractions 1, 2 et 3 par électrophorèse sur gel de polyacrylamide (SBS PAGE-coloration argentique) fait apparaître que la fraction 3 contient uniquement les deux facteurs bFGF et aFGF.

TABLEAU VI

| Résines | bFGF | | | aFGF | | |
|---|---|---|---|---|---|---|
| | Retenu et élué % | NaCl* M | Efficacité** | Retenu et élué % | NaCl* M | Efficacité** |
| Héparin Sepharose | 49 | 1,5 | 1 | 28 | 1,1 | 1 |
| PS Thréo | 11 | 1,4 | 0,55 | 2,2 | 0,5 | 0,07 |
| PS Ala | 7 | 1,2 | 0,36 | 3,3 | 1 | 0,12 |
| PS Meth | 15 | 1,35 | 0,79 | 8,2 | 1 | 0,29 |
| PS Phé | 13 | 1,5 | 0,68 | 15 | 1,1 | 0,54 |
| PSPro | 10 | 0,9 | 0,53 | 1 | 0,8 | 0,04 |
| PSHyPro | 8 | 0,7 | 0,16 | 4 | 1,0 | 0,12 |
| $PSSO_3Na$ | 14 | 1,5-2,0 | 0,29 | 14 | 1,15 | 0,48 |

\* Concentration maximum en NaCl de l'éluant nécessaire à l'élution du facteur de croissance après la préadsorption sur la résine

$$** \quad Efficacité = \frac{\% \text{ retenu et élué sur la résine}}{\% \text{ retenu et élué sur Heparin Sepharose}}$$

IV - Tests utilisant deux colonnes en série

Les résultats obtenus sur les résines PSThréo, PSAla et PSMéth ainsi que PSPhé, comparés à ceux obtenus dans les mêmes conditions, sur une résine Heparin Sepharose, sont indiqués dans le Tableau VI cité ci-dessus.

Ces résultats ont été obtenus à partir d'un même lot d'extrait acido-soluble des facteurs de croissance, auxquels ont été ajoutés, pour faciliter l'analyse, successivement les facteurs bFGF* et aFGF*. Ces résultats font apparaître deux ensembles de résines, un premier ensemble qui ne retient que les facteurs bFGF (PSThréo et PSAla) et un second qui retient les deux facteurs de croissance bFGF et aFGF (PSPhé et PSMéth).

Il est donc possible d'effectuer une séparation des deux facteurs purifiés en injectant l'extrait acido-soluble, ainsi que les facteurs bFGF* et aFGF* sur une colonne remplie de PSPhé, puis sur une colonne remplie de PSThréo. Cette double opération permet de retenir les deux facteurs sur la première colonne à une force ionique inférieure à 0,7M NaCl et d'effectuer alors leur élution successive sur la deuxième colonne.

L'élution sur la deuxième colonne PSThréo, pourra être effectuée en gradient de NaCl, depuis 0,7M NaCl jusqu'à 1,4M NaCl. Dans ces conditions, le aFGF, non retenu sur PSThréo, sera élué avant la bFGF qu'il est possible de récupérer en solution 1,4M NaCl.

V - Tests obtenus en chromatographie liquide haute performance

Une colonne en acier inoxydable, spécialement conçue pour travailler sous haute pression (de l'ordre de 10 à 20 bars), est remplie par percolation avec environ 1 g de la résine PSPhé. La colonne est connectée sur un appareil comprenant une pompe haute pression, un injecteur, un ou deux détecteurs et un système micro-ordinateur et intégrateur permettant d'obtenir les chromatogrammes des espèces injectées. L'opération est effectuée au plus en 30 minutes, contrairement au système basse pression qui exige plus de 10 heures. En outre, un système d'injection automatique permet d'effectuer une séparation préparative en procédant à une série d'injections de l'extrait acido-soluble, puis à une seule élution en gradient de concentration saline.

## Revendications

1. Application des résines de polymères ou copolymères sur lesquels sont fixés, de façon statistique, des groupes -$SO_3H$ et -$SO_3M$, où M représente un métal physiologiquement acceptable, comme phase stationnaire en chromatographie d'affinité pour la purification de facteurs de croissance de type FGF.

2. Application selon la revendication 1, caractérisée par le fait que, sur les polymères et copolymères, sont également fixés, de façon statistique, des groupes -$SO_2R$, où R représente un radical obtenu par enlèvement d'un atome d'hydrogène sur la fonction amine d'un acide aminé ou d'un dérivé d'acide aminé.

3. Application selon l'une des revendications 1 et 2, caractérisée par le fait que les groupes chimiques sont fixés de façon irréversible par réticulation.

4. Application selon l'une des revendications 1 à 3, caractérisée par le fait que le support polymère ou copolymères est choisi parmi le polystyrène, les polysaccharides, les polyesters cellulosiques, les polyéthers cellulosiques, l'acétate de polyvinyle, le chlorure de polyvinyle, le polyisoprène, le polybutadiène, et les copolymères du styrène, des esters cellulosiques, des éthers cellulosiques, de l'acétate de vinyle, du chlorure de vinyle, de l'isoprène et du butadiène.

5. Application selon la revendication 4, caractérisée par le fait que le polymère est le polystyrène.

6. Application selon l'une des revendications 1 à 5, caractérisée par le fait que les acides aminés sont des acides $\alpha$-aminés.

7. Application selon la revendication 6, caractérisée par le fait que les acides $\alpha$ aminés ou leurs dérivés sont choisis parmi l'alanine, la phénylalanine, les diacides aspartique et glutamique, la méthionine, la thréonine, la proline, l'hydroxyproline, la sérine et la lysine.

8. Procédé d'obtention d'un facteur de croissance aFGF ou bFGF purifié, ou d'un mélange de ces facteurs de croissance, caractérisé par le fait qu'il consiste à effectuer une chromatographie d'affinité, sur une résine telle que définie à l'une des revendications 1 à 7.

9. Procédé selon la revendication 8, caractérisé par le fait qu'on effectue une chromatographie sous haute pression.

10. Procédé selon l'une des revendications 8 et 9, caractérisé par le fait qu'on effectue une chromatographie d'affinité sur plusieurs résines en série.

11. Procédé selon l'une des revendications 8 à 10, caractérisé par le fait qu'on réalise une élution avec un tampon pH neutre d'une force ionique équivalente à celle d'une solution de NaCl O,15M à 2,5M.

## Claims

1. Application of polymer or copolymer resins onto which -$SO_3H$ and -$SO_3M$ groups are randomly bound, in which M denotes a physiologically acceptable metal, as the stationary phase in affinity chromatography for the purification of FGF type growth factors.

2. The application as claimed in claim 1 , characterized in that - $SO_2R$ groups are also randomly bound on the polymers and copolymers, groups in which R denotes a radical obtained by removal of a hydrogen atom from the amino group of an amino acid or an amino acid derivative.

3. The application as claimed in any one of claims 1 and 2 , characterized in that the chemical groups are irreversibly bound by crosslinking.

4. Application as claimed in any one of claims 1 to 3 , characterized in that the polymeric or copolymeric support is chosen from among polystyrene, polysaccharides, cellulose polyesters, cellulose polyethers, polyvinyl acetate, polyvinyl chloride, polyisoprene, polybutadiene, and the copolymers of styrene, cellulose esters, cellulose ethers, vinyl acetate, vinyl chloride, isoprene and butadiene.

5. The application as claimed in claim 4, characterized in that the polymer is polystyrene.

6. The application as claimed in any one of claims 1 to 5, characterized in that the amino acids are alpha-amino acids.

7. The application as claimed in claim 6, characterized in that the alpha-amino acids or their derivatives are chosen from among alanine, phenylalanine, aspartic and glutamic diacids, methionine, threonine, proline, hydroxyproline,serine and lysine.

8. A method for obtaining a purified aFGF or bFGF growth factor, or a mixture of these growth factors, characterized in that it consists in carrying out an affinity chromatography, on a resin such as defined in any one of claims 1 to 7.

9. The method claimed in claim 8, characterized in that a high-pressure chromatography is carried out.

10. The method claimed in any one of claims 8 and 9, characterized in that an affinity chromatography is carried out on several resins in series.

11. The method as claimed in any one of claims 8 to 10, characterized in that an elution is carried out with a neutral pH buffer having an ionic strength equivalent to that of a 0.15 M to 2.5M NaCl solution.

**Patentansprüche**

1. Verwendung von Harzen aus Polymeren oder Copolymeren, an die statistisch -SO$_3$H- und -SO$_3$M- Gruppen gebunden sind, wobei M ein physiologisch Verträgliches Metall darstellt, als stationäre Phase in der Affinitätschromatographie zur Reinigung von Wachstumsfaktoren des Typs FGF.

2. Verwendung nach Anspruch 1, dadurch gekennzeichnet, daß an die Polymere und Copolymere auch -SO$_2$R- Gruppen statistisch gebunden sind, in denen R einen Rest darstellt, der durch Abspaltung eines Wasserstoffatoms aus der Aminofunktion einer Aminosäure oder eines Derivats einer Aminosäure erhalten wurde.

3. Verwendung nach einem der Ansprüche 1 und 2, dadurch gekennzeichnet, daß die chemischen Gruppen irreversibel durch Vernetzung gebunden sind.

4. Verwendung nach einem der Ansprüche 1 bis 3, dadurch gekennzeichnet, daß der polymere oder copolymere Träger aus Polystyrol, Polysacchariden, Cellulosepolyestern, Cellulosepolyethern, Polyvinylacetat, Polyvinylchlorid, Polyisopren, Polybutadien und Copolymeren aus Styrol, Celluloseestern, Celluloseethern, Vinylacetat, Vinylchlorid, Isopren und Butadien ausgewählt ist.

5. Verwendung nach Anspruch 4, dadurch gekennzeichnet, daß das Polymer Polystyrol ist.

6. Verwendung nach einem der Ansprüche 1 bis 5, dadurch gekennzeichnet, daß die Aminosäuren α-Aminosäuren sind.

7. Verwendung nach Anspruch 6, dadurch gekennzeichnet, daß die α-Aminosäuren oder ihre Derivate aus Alanin, Phenylalanin, den zweibasigen Säuren Asparaginsäure und Glutaminsäure, Methionin, Threonin, Prolin, Hydroxyprolin, Serin und Lysin ausgewählt sind.

11

8. Verfahren zur Gewinnung eines gereinigten Wachstumsfaktors aFGF oder bFGF oder eines Gemisches aus diesen Wachstumsfaktoren, dadurch gekennzeichnet, daß eine Affinitätschromatographie auf einem Harz, das nach einem der Ansprüche 1 bis 7 definiert wurde, durchgeführt wird.

9. Verfahren nach Anspruch 8, dadurch gekennzeichnet, daß man eine Chromatographie unter hohem Druck durchführt.

10. Verfahren nach einem der Ansprüche 8 und 9, dadurch gekennzeichnet, daß man eine Affinitätschromatographie auf mehreren Harzen in Reihe durchführt.

11. Verfahren nach einem der Ansprüche 8 bis 10, dadurch gekennzeichnet, daß man mit einem pH-neutralen Puffer von einer Ionenstärke, die einer 0,15 M bis 2,5 M NaCl-Lösung äquivalent ist, eluiert.

FIG.1

FIG.2

aFGF: élution 1,15 M NaCl
bFGF: élution 2,15 M NaCl

FIG. 3a

FIG. 3b

bFGF

FIG. 3c

FIG. 4